# DEMANDE DE BREVET EUROPEEN

(11) **EP 1 013 260 A2**
(43) Date de publication de la demande: **28.06.2000**
(21) Numéro de dépôt: 99403075.7
(22) Date de dépôt: 08.12.1999
(51) Int. Cl.: A61K 7/13, C09B 62/00

(54) **Utilisation d'hydroxystilbènes pour la teinture, composition prête à l'emploi les contenant et procédé de teinture**

(30) Priorité: 22.12.1998 FR 9816258
(71) Demandeur: L'OREAL, 75008 Paris (FR)
(72) Inventeur: Pruche, Francis, 75018 Paris (FR); Saint Leger, Didier, 92400 Courbevoie (FR); Bernard, Bruno, 92200 Neuilly S/Seine (FR)
(74) Mandataire: Casalonga, Axel

(57) **Abrégé**

L'invention est relative à l'utilisation d'hydroxystilbènes pour la teinture, à des compositions tinctoriales prêtes à l'emploi les comprenant, à un procédé de teinture les mettant en oeuvre ainsi qu'à un dispositif à plusieurs compartiments renfermant les compositions mises en oeuvre dans les procédés de l'invention.

## Description

La présente invention est relative à l'utilisation d'hydroxystilbènes, à des compositions tinctoriales prêtes à l'emploi les contenant, aux procédés de teinture les mettant en oeuvre et à un dispositif à plusieurs compartiments utilisé pour la mise en oeuvre desdits procédés de teinture.

Il est bien connu d'utiliser dans les compositions capillaires de coloration des colorants dits "d'oxydation". Ces colorants présentent l'avantage de conduire à des nuances plus couvrantes et plus tenaces que celles obtenues par l'utilisation de colorants dits "directs". Néanmoins, les colorants d'oxydation actuellement utilisés présentent l'inconvénient de ne pas être totalement inoffensifs et comportent ainsi des risques potentiels d'allergies.

La demanderesse a recherché des colorants d'oxydation qui ne présentent pas ces inconvénients et qui permettent de conférer aux cheveux une coloration ayant une résistance satisfaisante à la lumière, aux lavages, aux intempéries, à la transpiration et aux différents traitements que peuvent subir les cheveux. De plus, on recherche également des colorants oxydants qui peuvent être utilisés dans le cadre de colorations enzymatiques et qui peuvent ainsi permettre d'éviter l'utilisation d'agents oxydants tels que le peroxyde d'hydrogène, qui peut être à l'origine d'une dégradation non négligeable des fibres kératiniques.

La demande japonaise n° 64-38009 décrit l'utilisation d'hydroxystilbènes comme agent inhibiteur de tyrosinase et divulgue des compositions dépigmentantes contenant des hydroxystilbènes.

La demanderesse vient de découvrir, ce qui fait l'objet de l'invention, que l'utilisation d'hydroxystilbènes comme précurseur de colorant d'oxydation dans des compositions tinctoriales permettait d'obtenir un large éventail de colorations, lesdites colorations présentant une résistance particulièrement remarquable à la lumière, aux lavages, aux intempéries et à la transpiration.

L'invention a ainsi pour objet l'utilisation d'hydroxystilbène pour la teinture, et particulièrement pour la teinture des matières kératiniques.

Un autre objet de l'invention est constitué par des compositions tinctoriales prêtes à l'emploi contenant au moins un hydroxystilbène et un agent oxydant.

Un objet de l'invention concerne aussi un procédé de teinture d'un support, et en particulier de matières kératiniques, mettant en oeuvre de telles compositions.

Un autre objet de l'invention porte sur un dispositif à plusieurs compartiments ou kits de teinture renfermant les différents composés utilisés pour les teintures selon l'invention pour la teinture.

La présente invention a donc pour objet essentiel l'utilisation d'hydroxystilbène de formule (I) suivante: 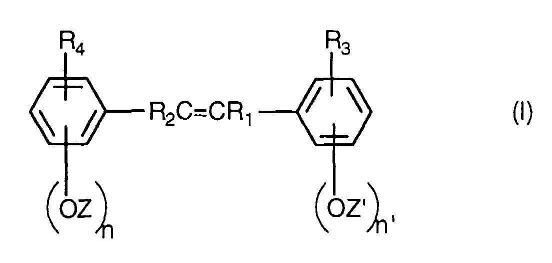 dans laquelle:
n désigne un nombre entier de 2 à 4,
n' désigne un nombre entier de 0 à 4,
R₁ et R₂, identiques ou différents, désignent un atome d'hydrogène, un atome d'halogène, un radical alkyle, alkoxy, carboxy ou acyle;
R₃ et R₄, identiques ou différents, désignent un atome d'hydrogène, un atome d'halogène, un radical alkyle, alkoxy, carboxy ou acyle;
Z et Z', identiques ou différents, représentent un atome d'hydrogène ou un radical glycosyl;
ou l'un de leurs sels d'addition avec un acide.

Dans la formule (I) ci-dessus, les radicaux alkyle, alkoxy ou acyle peuvent être linéaires ou ramifiés.

Les groupements alkyle désignent notamment des groupements de 1 à 20 atomes de carbone, comme par exemple les groupements éthyle, méthyle, propyle, isopropyle, n-propyle, butyle, n-butyle, tert.butyle, pentyle, n-pentyl, isopentyl, n-hexyle, isohexyle, octyle, nonyle, décyle, indécyle, pentadécyle. Préférentiellement, ces groupements alkyles désignent un groupement de 1 à 6 atomes de carbone.

Les groupement alkoxy désignent les groupements -O-R, R représentant un groupement alkyle tel que défini ci-avant.

Les groupements acyle désignent les groupements -COR, R représentant un groupement alkyle tel que défini ci-dessus.

Le radical glycosyl représente un radical dérivé d'un glycose, c'est à dire d'un monosaccharide ou sucre simple, tel que l'arabinose, le glucose ou le fructose. Le radical glycosyl désigne préférentiellement un radical glucosyl.

Les hydroxystilbènes de formule (I) peuvent être utilisés pour la teinture de supports très divers, tels que du coton, de la cellulose, des matières plastiques, etc. En effet, il a été remarqué que l'utilisation de ces précurseurs de colorants permettaient d'obtenir des colorants possédant une forte affinité pour leur support.

Dans un mode de réalisation préférée de l'invention, les hydroxystilbènes sont utilisés pour la teinture des matières kératiniques telles que la peau ou les fibres kératiniques comme les cheveux ou les ongles.

Dans une forme de réalisation préférée de l'invention, les composés de formule (I) sont choisis parmi ceux pour lesquels R₁ et R₂ désignent un atome d'hydrogène et R₃ et R₄, identiques ou différents, désignent un atome d'hydrogène, un radical alkyle en C₁-C₄, alkoxy en C₁-C₄ ou carboxy.

Encore plus préférentiellement, les hydroxystilbènes de formule (I) sont choisis parmi les composés suivants:
- le 4'-methoxy-3,3',5-stilbenetriol-3-glucoside ou rhapontin
- le trans-1-(3'-carboxy-4'-hydroxyphenyl)-2-(2',5'-dihydroxyphenyl)éthane,
- le 3,5-dihydroxy-4'-methoxy stilbène 3-O-béta-D-glucoside ou déoxyrhapontin,
- le trans-3,4',5-trihydroxystilbène ou resvératrol,
- le 4',5-dihydroxystilbène-3-O-béta-D-glucoside
- le 3,3',4,5'-tétrahydroxystilbène ou picestannol,
- le 3,5 dihydroxy-4'-bromostilbène,
- le 2,3,5,4' tétrahydroxystilbène-2-O-béta-D-Glucoside, et
- le 3,5,3'-trihydroxy-4'-methoxy stilbène 5-O-béta-D-glucoside;
ou l'un de leurs sels d'addition avec un acide.

Dans le cadre de la présente invention, les sels d'addition avec un acide sont notamment choisis parmi les chlorhydrates, les bromhydrates, les sulfates, les citrates, les succinates, les tartrates, les lactates et les acétates.

Un autre objet de l'invention concerne les compositions tinctoriales prêtes à l'emploi comprenant dans un milieu approprié pour la teinture au moins un composé de formule (I) tel que défini ci-dessus et au moins un agent oxydant.

L'agent oxydant pourra notamment être choisi parmi les enzymes, le peroxyde d'hydrogène, le peroxyde d'urée, les persels et peracides, les sels métalliques comme par exemple les sels de cuivre, les quinones et les nitrites.

Les enzymes peuvent notamment être choisies parmi les pyranose oxydases, les glucose oxydases, les glycérol oxydases, les lactate oxydases, les pyruvate oxydases, les uricases, les choline oxydases, les sarcosine oxydases, les bilirubines oxydases, les laccases, les tyrosinases, les péroxydases, les catalases ou parmi des extraits végétaux ou animaux contenant les enzymes précitées, en présence éventuelle d'un donneur (ou substrat) nécessaire au fonctionnement desdites enzymes.

Lorsque les enzymes sont utilisées comme agents oxydants, et notamment la tyrosinase, les compositions tinctoriales prêtes à l'emploi de l'invention peuvent ainsi contenir en outre de la L-tyrosine; des dérivés de pyrimidine 3-oxyde, substitués en 6, notamment ceux décrits dans FR 9611316, de la L-DOPA; de la dopamine; des peptides avec des résidus de L-tyrosine, L-DOPA et/ou Dopamine; de l'acide caféique; de l'acide coumarique; de l'acide chlorogénique; de l'acide ellagique; des catéchines; des OPC ou des extraits végétaux ou animaux contenant ces composés précités.

Les enzymes utilisées selon l'invention peuvent être d'origine animale, microbiologique (bactérienne, fongique ou virale) ou synthétique (obtenue par synthèse chimique ou biotechnologique).

La ou les enzymes peuvent être utilisées sous forme cristalline pure ou sous une forme diluée dans un diluant inerte pour ladite enzyme.

A titre d'exemple d'uricases, on peut notamment citer l'uricase extraite de foie de sanglier, l'uricase d'Arthrobacter globiformis, ainsi que l'uricase d'Aspergillus flavus.

A titre d'exemple de sources de choline oxydase, on peut notamment citer le foie de rat, les bactéries telles que Arthrobacter globiformis, Achromobacter cholinophagum ou Alcaligenes, et les champignons tels que Cylindrocarpon didynum.

A titre d'exemple de sources de sarcosine oxydase, on peut notamment citer les bactéries telles que Arthrobacter et en particulier Arthrobacter ureafaciens et Arthrobacter globiformis, Streptomyces, Bacillus, Pseudomonas, Corynebacterium ou Alcaligenes tels que par exemple Alcaligenes denitrificans, et les champignons tels que Cylindrocarpon didynum.

A titre d'exemple de sources de bilirubine oxydase, on peut notamment citer la muqueuse intestinale et le foie de rat, les bactéries telles que Myrothecium verucania, Myrothecium cinctum, et Myrothecium roridum.

Parmi les laccases d'origine végétale utilisables selon l'invention, on peut citer les laccases produites par des végétaux effectuant la synthèse chlorophylienne telles que celles indiquées dans la demande de brevet FR-A-2 694 018.

On peut notamment citer les laccases extraites d'Anacardiacées, de Podocarpacées, de Rosmarinus off., de Solanum tuberosum, dIris sp., de Coffea sp., de Daucus carrota, de Vinca minor, de Persea americana, de Catharenthus roseus, de Musa sp., de Malus pumila, de Gingko biloba, et de Monotropa hypopithys (sucepin).

Parmi les laccases d'origine microbienne (notamment fongique), ou obtenues par biotechnologie utilisables selon l'invention, on peut citer les laccases de Polyporus versicolor, de Rhizoctonia praticola et de Rhus vernicifera telles que décrites par exemples dans les demandes de brevet FR-A-2 112 549 et EPA504005; les laccases décrites dans les demandes de brevet WO95/07988, WO95/33836, WO95/33837, WO96/00290, WO97/19998 et WO97/19999, dont le contenu fait partie intégrante de la présente description comme par exemple les laccases de Scytalidium, de Polyporus pinsitus, de Myceliophthora thermophila, de Rhizoctonia solani, de Pyricularia orizae, et leurs variantes.

On choisira plus préférentiellement les laccases d'origine microbienne ou celles obtenues par biotechnologie.

Dans une forme de réalisation particulièrement préférée de l'invention, l'enzyme utilisée correspond à la tyrosinase. Par tyrosinase, il faut entendre dans la présente invention, toute enzyme présentant une activité tyrosinase, cette enzyme pouvant présenter d'autres activités enzymatiques. L'activité tyrisonase peut se définir comme l'activité enzymatique qui catalyse l'oxydation de la tyrosinase pour conduire à la formation du précurseur de mélanine: la Dopaquinone.

A titre d'exemple de sources de tyrosinase, on peut notamment citer la pomme de terre, les champignons, les micro-organismes tels que Neurospora crassa, etc...

Dans les compositions prêtes à l'emploi selon l'invention, le ou les composés de formule (I) représentent de 0,01 à 10% en poids du poids total de la composition tinctoriale prête à l'emploi, et préférentiellement de 0,1 à 5% en poids du poids total de la composition tinctoriale prête à l'emploi.

L'agent oxydant peut quant à lui représenter de 0,001 à 25% en poids du poids total de la composition tinctoriale prête à l'emploi et préférentiellement de 0,1 à 10% en poids du poids total de la composition tinctoriale prête à l'emploi.

La composition tinctoriale prête à l'emploi conforme à l'invention peut en outre renfermer un ou plusieurs précurseurs de colorants d'oxydation différents de ceux de formule (I), c'est à dire une ou plusieurs bases d'oxydation, et/ou un ou plusieurs coupleurs.

Lorsqu'elles sont présentes, la ou les bases d'oxydation représentent de préférence de 0,0005 à 12% en poids environ du poids total de la composition tinctoriale prête à l'emploi conforme à l'invention, et encore plus préférentiellement de 0,005 à 8% en poids environ de ce poids.

Lorsqu'ils sont présents, le ou les coupleurs représentent de préférence de 0,0001 à 10% en poids environ du poids total de la composition tinctoriale prête à l'emploi et encore plus préférentiellement de 0,005 à 8% en poids environ de ce poids.

La composition tinctoriale prête à l'emploi selon l'invention peut aussi contenir un ou plusieurs colorants directs qui peuvent notamment être choisis parmi les colorants azoïques, anthraquinoniques et les dérivés nitrés de la série benzénique, notamment pour modifier les nuances ou les enrichir en reflets.

Lorsque les compositions de l'invention sont utilisées pour la teinture de la peau, elles peuvent contenir en outre de la DHA.

Le milieu approprié pour la teinture est généralement constitué par de l'eau ou par un mélange d'eau et d'au moins un solvant organique pour solubiliser les composés qui ne seraient pas suffisamment solubles dans l'eau.

Les solvants peuvent être présents dans des proportions de préférence comprises entre 1 et 40% en poids environ par rapport au poids total de la composition tinctoriale, et encore plus préférentiellement entre 5 et 30% en poids environ.

Le pH des compositions prêtes à l'emploi de l'invention pourra être adapté en fonction de l'agent oxydant utilisé et de manière à ce que les propriétés tinctoriales de la composition ne soient pas altérées. Les pH devront notamment être adaptés de manière à ne pas dénaturer les enzymes lorsque ces dernières sont utilisées comme agents oxydants. Ainsi, les compositions de la présente invention pourront présenter un pH compris entre 3 et 12 et préférentiellement un pH inférieur à 8.

Ce pH peut être ajusté à la valeur désirée au moyen d'agents acidifiants ou alcalinisants habituellement utilisés en teinture.

La composition tinctoriale conforme à l'invention peut également renfermer divers adjuvants utilisés classiquement dans les compositions pour la teinture, et notamment pour la teinture des cheveux, tels que des agents tensio-actifs, des polymères, des agents épaississants minéraux ou organiques, des agents antioxydants, des agents de pénétration, des agents séquestrants, des parfums, des tampons, des agents dispersants ou des agents de conditionnement.

Bien entendu, l'homme de l'art veillera à choisir ce ou ces éventuels composés complémentaires précités de manière telle que les propriétés avantageuses attachées intrinsèquement à la composition de teinture conforme à l'invention ne soient pas, ou substantiellement pas, altérées par la ou les adjonctions envisagées.

La composition tinctoriale prête à l'emploi selon l'invention peut se présenter sous des formes diverses, telles que sous forme de liquides, de crèmes, de gels, ou sous toute autre forme appropriée pour réaliser une teinture et notamment des matières kératiniques.

Un autre objet de l'invention concerne un procédé de teinture dans lequel on applique sur un support au moins une composition tinctoriale telle que définie ci-avant en un temps suffisant pour développer la coloration désirée.

Préférentiellement, le support utilisé dans ce procédé de teinture consiste en des matières kératiniques. Dans ce cas, le temps nécessaire pour développer la coloration pourra être de l'ordre de 1 minute à 1 heure et plus précisément de 5 à 40 minutes. Après cette application sur les matières kératiniques, on rince, on lave éventuellement au shampooing, on rince à nouveau et on sèche.

Dans une autre forme de réalisation du procédé de l'invention, ledit procédé comporte une étape préliminaire consistant à stocker sous forme séparée, d'une part, une composition (A) comprenant dans un milieu approprié pour la teinture, au moins un composé de formule (I) tel que défini ci-dessus et, d'autre part, une composition (B) renfermant, dans un milieu approprié pour la teinture, au moins un agent oxydant tel que défini ci-avant, puis à procéder à leur mélange au moment de l'emploi avant d'appliquer ce mélange sur le support.

La présente invention concerne en outre un dispositif à plusieurs compartiments ou "kit" de teinture comportant un premier compartiment renfermant la composition (A) définie ci-dessus et un second compartiment renfermant la composition (B) définie ci-avant.

Lorsque l'agent oxydant de la composition (B) correspond à une enzyme, cette dernière peut être sous forme de poudre ou immobilisée, c'est à dire fixée sur une matrice.

Ces dispositifs peuvent être équipés d'un moyen permettant de délivrer sur les cheveux le mélange souhaité, tel que les dispositifs décrits dans le brevet FR-2 586 913.

Les exemples qui suivent sont destinés à illustrer l'invention sans pour autant en limiter la portée.

On a préparé les compositions tinctoriales conformes à l'invention suivantes:

### EXEMPLES DE TEINTURE 1 à 4

| **Exemples** | **1** | **2** | **3** | **4** |
|---|---|---|---|---|
| Resvératrol (Composé de formule (I)) | 5,26 mmole | 5,26 mmole | 5,26 mmole | 5,26 mmole |
| 5,6-dihydroxy indoline | - | 0,173 mmole | - | - |
| 2,4-diamino phénoxyéthanol, 2HCl | - | - | 0,173 mmole | - |
| 5,6-dihydroxy indole | - | - | - | 0.173 mmole |
| Acide caféique | 0,173 mmole | - | - | - |
| Laccase | 0,002 g | 0,002 g | 0,002 g | 0,002 g |
| Tampon Phosphate pH 7,2 qsp | 100 ml | 100 ml | 100 ml | 100 ml |

### EXEMPLES DE TEINTURE 5 à 9

| **Exemples** | **5** | **6** | **7** | **8** | **9** |
|---|---|---|---|---|---|
| Resvératrol (Composé de formule (I)) | 5,26 mmole | 5,26 mmole | 5,26 mmole | 5,26 mmole | 5,26 mmole |
| 2,4-diamino phénoxyéthanol, 2HCl | - | - | - | 0,173 mmole | - |
| 5,6-dihydroxy indole | - | - | - | - | 0,173 mmole |
| Paraphénylènediamine | 0,173 mmole | - | - | - | - |
| Acide caféique | - | - | 0,173 mmole | - | - |
| Laccase Laccase | 0,002 g | - | - | - | - |
| Tyrosinase | - | 0,068 g | 0,068 g | 0,068 g | 0,068 g |
| Tampon Phosphate pH 7,2 qsp | 100 ml | 100 ml | 100 ml | 100 ml | 100 ml |

Chacune des compositions tinctoriales ci-dessus a été appliquée sur des mèches de cheveux gris naturels à 90% de blancs pendant 30 minutes à 37°C.

Les mèches ont ensuite été rincées, lavées au shampooing, rincées à nouveau plus séchées.

Les cheveux ont été teints dans une nuance figurant dans le tableau ci-après:

| **EXEMPLES** | **NUANCE OBTENUE** |
|---|---|
| **1** | Blond très clair doré |
| **2** | Blond clair cendré |
| **3** | Blond clair cendré légèrement mat |
| **4** | Blond clair cendré légèrement doré mat |
| **5** | Blond mat légèrement doré |
| **6** | Blond clair doré |
| **7** | Blond clair doré |
| **8** | Blond très clair cendré légèrement mat |
| **9** | Blond cendré |

## Revendications

1. Utilisation pour la teinture d'hydroxystilbène de formule (I) suivante: 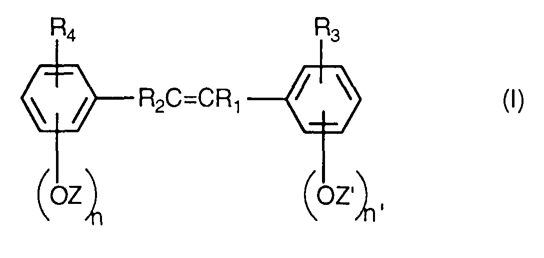 dans laquelle:
n désigne un nombre entier de 2 à 4,
n' désigne un nombre entier de 0 à 4,
R₁ et R₂, identiques ou différents, désignent un atome d'hydrogène, un atome d'halogène, un radical alkyle, alkoxy, carboxy ou acyle;
R₃ et R₄, identiques ou différents, désignent un atome d'hydrogène, un atome d'halogène, un radical alkyle, alkoxy, carboxy ou acyle;
Z et Z', identiques ou différents, représentent un atome d'hydrogène ou un radical glycosyl;
ou l'un de leurs sels d'addition avec un acide.

2. Utilisation selon la revendication 1, pour la teinture des matières kératiniques.

3. Utilisation selon la revendication 1 ou 2, caractérisée en ce que R₁ et R₂ désignent un atome d'hydrogène, R₃ et R₄, identiques ou différents, désignent un atome d'hydrogène, un radical alkyle en C₁-C₄, alkoxy en C₁-C₄ ou carboxy.

4. Utilisation selon l'une quelconque des revendications 1 à 3, caractérisée par le fait que les hydroxystilbènes de formule (I) sont choisis parmi les composés suivants:
- le 4'-methoxy-3,3',5-stilbènetriol-3-glucoside,
- le trans-1-(3'-carboxy-4'-hydroxyphenyl)-2-(2",5"-dihydroxyphenyl)éthane
- le 3,5-dihydroxy-4'-methoxy stilbène 3-O-béta-D-glucoside,
- le trans-3,4',5-trihydroxystilbène,
- le 4',5-dihydroxystilbène-3-O-béta-D-glucoside,
- le 3,3',4,5'-tétrahydroxystilbène,
- le 3,5 dihydroxy-4'-bromostilbène,
- le 2,3,5,4' tétrahydroxystilbène-2-O-béta-D-Glucoside, et
- le 3,5,3'-trihydroxy-4'-methoxy stilbène 5-O-béta-D-glucoside;
ou l'un de leurs sels d'addition avec un acide.

5. Utilisation selon l'une quelconque des revendications 1 à 4, caractérisée par le fait que les sels d'addition avec un acide sont choisis parmi les chlorhydrates, les bromhydrates, les sulfates, les citrates, les succinates, les tartrates, les lactates et les acétates.

6. Composition tinctoriale prête à l'emploi, caractérisée par le fait qu'elle comprend, dans un milieu approprié pour la teinture, au moins un composé de formule (I) tel que défini à l'une quelconque des revendications 1 à 5 et au moins un agent oxydant.

7. Composition selon la revendication 6, caractérisée par le fait que l'agent oxydant est choisi parmi les enzymes, le peroxyde d'hydrogène, le peroxyde d'urée, les persels et peracides, les sels métalliques, les quinones et les nitrites.

8. Composition selon la revendication 7, caractérisée par le fait que les enzymes sont choisies parmi les pyranose oxydases, les glucose oxydases, les glycérol oxydases, les lactate oxydases, les pyruvate oxydases, les uricases, les cholines oxydases, les laccases, les sarcosine oxydases, les bilirubine oxydases, les tyrosinases, les péroxydases, les catalases ou parmi des extraits végétaux ou animaux contenant les enzymes précitées, en présence éventuelle d'un donneur (ou substrat) nécessaire au fonctionnement desdites enzymes.

9. Composition selon la revendication 8, caractérisée par le fait que l'enzyme correspond à la tyrosinase.

10. Composition selon l'une quelconque des revendications 6 à 9, caractérisée par le fait que le ou les composés de formule (I) représentent de 0,01 à 10% en poids du poids total de la composition tinctoriale prête à l'emploi.

11. Composition selon la revendication 10, caractérisée par le fait que le ou les composés de formule (I) représentent de 0,1 à 5% en poids du poids total de la composition tinctoriale prête à l'emploi.

12. Composition selon l'une quelconque des revendications 6 à 11, caractérisée par le fait que l'agent oxydant représente de 0,001 à 25% en poids du poids total de la composition tinctoriale prête à l'emploi.

13. Composition selon la revendication 12, caractérisée par le fait que l'agent oxydant représente de 0,1 à 10% en poids du poids total de la composition tinctoriale prête à l'emploi.

14. Composition selon l'une quelconque des revendications 6 à 13, caractérisée par le fait qu'elle renferme une ou plusieurs bases d'oxydation différentes des composés de formule (I) et/ou un ou plusieurs coupleurs.

15. Composition selon la revendication 14, caractérisée par le fait que la ou les bases d'oxydation représentent de 0,0005 à 12% en poids du poids total de la composition tinctoriale prête à l'emploi et que le ou les coupleurs représentent de 0,0001 à 10% en poids du poids total de la composition tinctoriale prête à l'emploi.

16. Composition selon la revendication 15, caractérisée par le fait que la ou les bases d'oxydation représentent de 0,005 à 8% en poids du poids total de la composition tinctoriale et que le ou les coupleurs représentent de 0,005 à 8% en poids du poids total de la composition tinctoriale.

17. Composition selon l'une quelconque des revendications 6 à 16, caractérisée par le fait qu'elle contient en outre des colorants directs et/ou autooxydables choisis parmi les colorants azoïques, anthraquinoniques et les dérivés nitrés de la série benzénique.

18. Composition selon l'une quelconque des revendications 6 à 17, caractérisée par le fait que le milieu approprié pour la teinture est constitué par de l'eau ou par un mélange d'eau et d'au moins un solvant organique.

19. Composition selon l'une quelconque des revendications 6 à 18, caractérisée par le fait qu'elle présente un pH compris entre 3 et 12.

20. Composition selon l'une quelconque des revendications 6 à 19, caractérisée par le fait qu'elle contient en outre des agents tensio-actifs, des polymères, des agents épaississants minéraux ou organiques, des agents antioxydants, des agents de pénétration, des agents séquestrants, des parfums, des tampons, des agents dispersants ou des agents de conditionnement.

21. Composition selon l'une quelconque des revendications 9 à 20, caractérisée par le fait qu'elle contient en outre de la L-tyrosine; un dérivé de pyrimidine 3-oxyde, substitué en 6; de la L-DOPA; de la dopamine; des peptides avec des résidus de L-tyrosine, L-DOPA et/ou de dopamine; de l'acide caféique; de l'acide coumarique; de l'acide chlorogénique; de l'acide ellagique; des catéchines; des OPC ou des extraits végétaux ou animaux contenant ces composés précités.

22. Composition tinctoriale selon l'une quelconque des revendications 6 à 21, caractérisée en ce qu'elle se présente sous forme de liquides, de crèmes, de gels, ou sous toute autre forme appropriée pour réaliser une teinture.

23. Procédé de teinture, caractérisé par le fait que l'on applique sur un support au moins une composition tinctoriale telle que définie selon l'une quelconque des revendications 6 à 22 pendant un temps suffisant pour développer la coloration désirée.

24. Procédé selon la revendication 23, caractérisé par le fait qu'il comporte une étape préliminaire consistant à stocker sous forme séparée, d'une part, une composition (A) comprenant dans un milieu approprié pour la teinture, au moins un composé de formule (I) tel que défini à l'une quelconque des revendications 1 à 5 et, d'autre part, une composition (B) renfermant, dans un milieu approprié pour la teinture, au moins un agent oxydant tel que défini à l'une quelconque des revendications 7 à 9, puis à procéder à leur mélange au moment de l'emploi avant d'appliquer ce mélange sur le support.

25. Dispositif à plusieurs compartiments ou "kit" de teinture, caractérisé par le fait qu'il comporte un premier compartiment renfermant la composition (A) telle que définie à la revendication 24 et un second compartiment renfermant la composition (B) telle que définie à la revendication 24.
